(19) Europäisches Patentamt
European Patent Office
Office européen des brevets

(11) EP 1 326 638 B1

(12) **EUROPEAN PATENT SPECIFICATION**

(45) Date of publication and mention of the grant of the patent:
**28.11.2007 Bulletin 2007/48**

(21) Application number: **01987671.3**

(22) Date of filing: **16.10.2001**

(51) Int Cl.:
***A61K 39/39*** *(2006.01)*

(86) International application number:
**PCT/EP2001/011984**

(87) International publication number:
**WO 2002/032450 (25.04.2002 Gazette 2002/17)**

(54) **Vaccines against cancers**

Impfstoffe gegen Krebskrankheiten

Vaccins contre les cancers

(84) Designated Contracting States:
**AT BE CH CY DE DK ES FI FR GB GR IE IT LI LU MC NL PT SE TR**
Designated Extension States:
**SI**

(30) Priority: **18.10.2000 GB 0025573**
**18.10.2000 GB 0025574**
**18.10.2000 US 690921**

(43) Date of publication of application:
**16.07.2003 Bulletin 2003/29**

(73) Proprietor: **GlaxoSmithKline Biologicals s.a.**
**1330 Rixensart (BE)**

(72) Inventors:
- **GARCON, Nathalie,**
**GlaxoSmithKline Biologicals sa**
**B-1330 Rixensart (BE)**
- **GERARD, Catherine Marie, Ghislaine,**
**SmithKline Beecham**
**B-1330 Rixensart (BE)**
- **STEPHENNE, Jean,**
**SmithKline Beecham Biologicals SA**
**B-1330 Rixensart (BE)**

(74) Representative: **Dalton, Marcus Jonathan William**
**GlaxoSmithKline**
**Corporate Intellectual Property (CN9.25.1)**
**980 Great West Road**
**Brentford, Middlesex TW8 9GS (GB)**

(56) References cited:
**WO-A-00/09159**
**WO-A-00/44899**
**WO-A-00/62800**
**WO-A-01/51083**
**WO-A-99/40188**
**WO-A-99/61056**
**WO-A-02/069369**

Remarks:
The file contains technical information submitted after the application was filed and not included in this specification

EP 1 326 638 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**SEQUENCE LISTING**

<110> SmithKline Beecham Biologicals sa

<120> Vaccines

<130> B45245

<160> 5

<170> FastSEQ for Windows Version 4.0

<210> 1
<211> 20
<212> DNA
<213> Artificial sequence
<220>
<223> Synthetic oligonucleotide

<400> 1
tccatgacgt tcctgacgtt 20

<210> 2
<211> 18
<212> DNA
<213> Artificial sequence
<220>
<223> Synthetic oligonucleotide

<400> 2
tctcccagcg tgcgccat 18

<210> 3
<211> 30
<212> DNA
<213> Artificial sequence
<220>
<223> Synthetic oligonucleotide

<400> 3
accgatgacg tcgccggtga cggcaccacg 30

<210> 4
<211> 24

<212> DNA
<213> Artificial sequence
<220>
<223> Synthetic oligonucleotide

<400> 4
tcgtcgtttt gtcgttttgt cgtt 24

<210> 5
<211> 20
<212> DNA
<213> Artificial sequence
<220>
<223> Synthetic oligonucleotide

<400> 5
tccatgacgt tcctgatgct 20

## Description

The present invention relates to a novel formulation comprising a combination of a cancer antigen or derivative thereof and a combined adjuvant composition comprising a lipopolysaccharide, an immunostimulatory oligonucleotide and a saponin as defined in claim 1. The antigen is preferably a Her 2 neu derivative or P501S and consequently the formulations of the invention have utility in the treatment and prevention of humans expressing such antigens.

Despite enormous investments of financial and human resources, cancer remains one of the major causes of death. For example, cancer is the leading cause of death in women between the ages of 35 and 74. Breast cancer is the most common malignancy in women and the incidence for developing breast cancer is on the rise. It is estimated that one in nine women will be diagnosed with the disease. Standard approaches to cure breast cancer have centered around a combination of surgery, radiation and chemotherapy. These approaches have resulted in some dramatic successes in certain malignancies. However, breast cancer is most often incurable, when diagnosed beyond a certain stage. Alternative approaches to early diagnosis and therapy are necessary.

Immunostimulatory oligonucleotides containing unmethylated CpG dinucleotides ("CpG") and are known in the art as being adjuvants when administered by both systemic and mucosal routes (WO 96/02555, EP 468520, Davis et al., J.Immunol, 1998, 160(2):870-876; McCluskie and Davis, J.Immunol., 1998, 161(9):4463-6). CpG is an abbreviation for cytosine-guanosine dinucleotide motifs present in DNA. Historically, it was observed that the DNA fraction of BCG could exert an anti-tumour effect. In further studies, synthetic oligonucleotides derived from BCG gene sequences were shown to be capable of inducing immunostimulatory effects (both in vitro and in vivo). The authors of these studies concluded that certain palindromic sequences, including a central CG motif, carried this activity. The central role of the CG motif in immunostimulation was later elucidated in a publication by Krieg, Nature 374, p546 1995. Detailed analysis has shown that the CG motif has to be in a certain sequence context, and that such sequences are common in bacterial DNA but are rare in vertebrate DNA. The immunostimulatory sequence is often: Purine, Purine, C, G, pyrimidine, pyrimidine; wherein the dinucleotide CG motif is not methylated, but other unmethylated CpG sequences are known to be immunostimulatory and may be used in the present invention.

In certain combinations of the six nucleotides a palindromic sequence is present. Several of these motifs, either as repeats of one motif or a combination of different motifs, can be present in the same oligonucleotide. The presence of one or more of these immunostimulatory sequence containing oligonucleotides can activate various immune subsets, including natural killer cells (which produce interferon y and have cytolytic activity) and macrophages (Wooldrige et al Vol 89 (no. 8), 1977). Although other unmethylated CpG containing sequences not having this consensus sequence have now been shown to be immunomodulatory.

CpG when formulated into vaccines, is generally administered in free solution together with free antigen (WO 96/02555; McCluskie and Davis, *supra*) or covalently conjugated to an antigen (PCT Publication No. WO 98/16247), or formulated with a carrier such as aluminium hydroxide ((Hepatitis surface antigen) Davis *et al. supra* ; Brazolot-Millan et al., Proc.Natl.Acad.Sci., USA, 1998, 95(26), 15553-8).

The adjuvant combinations of the present invention include, in preferred embodiments, at least one enterobacterial lipopolysaccharide derived adjuvant.

It has long been known that enterobacterial lipopolysaccharide (LPS) is a potent stimulator of the immune system, although its use in adjuvants has been curtailed by its toxic effects. A non-toxic derivative of LPS, monophosphoryl lipid A (MPL), produced by removal of the core carbohydrate group and the phosphate from the reducing-end glucosamine, has been described by Ribi et al (1986, Immunology and Immunopharmacology of bacterial endotoxins, Plenum Publ. Corp., NY, p407-419) and has the following structure:

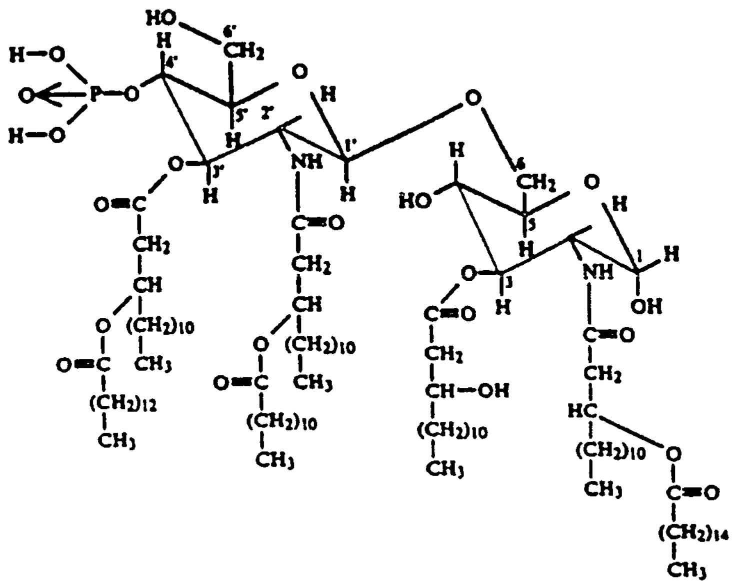

A further detoxified version of MPL results from the removal of the acyl chain from the 3-position of the disaccharide backbone, and is called 3-O-Deacylated monophosphoryl lipid A (3D-MPL). It can be purified and prepared by the methods taught in GB 2122204B, which reference also discloses the preparation of diphosphoryl lipid A, and 3-O-deacylated variants thereof. A preferred form of 3D-MPL is in the form of an emulsion having a small particle size less than 0.2μm in diameter, and its method of manufacture is disclosed in WO 94/21292. Aqueous formulations comprising monophosphoryl lipid A and a surfactant have been described in WO 98/43670A2.

The bacterial lipopolysaccharide derived adjuvants to be formulated in the adjuvant combinations of the present invention may be purified and processed from bacterial sources, or alternatively they may be synthetic. For example, purified monophosphoryl lipid A is described in Ribi et al 1986 (supra), and 3-O-Deacylated monophosphoryl or diphosphoryl lipid A derived from *Salmonella sp.* is described in GB 2220211 and US 4912094. Other purified and synthetic lipopolysaccharides have been described (WO 98/01139; US 6,005,099 and EP 0 729 473 B1; Hilgers et al., 1986, Int.Arch.Allergy.Immunol., 79(4):392-6; Hilgers et al., 1987, Immunology, 60(1):141-6; and EP 0 549 074 B1). Particularly preferred bacterial lipopolysaccharide adjuvants are 3D-MPL and the β(1-6) glucosamine disaccharides described in US 6,005,099 and EP 0 729 473 B1.

Accordingly, the LPS derivatives that may be used in the present invention are DIPHOSPHORYL LIPIDA, MPL or 3D-MPL.

One disaccharide adjuvant is a purified or synthetic lipid A of the following formula:

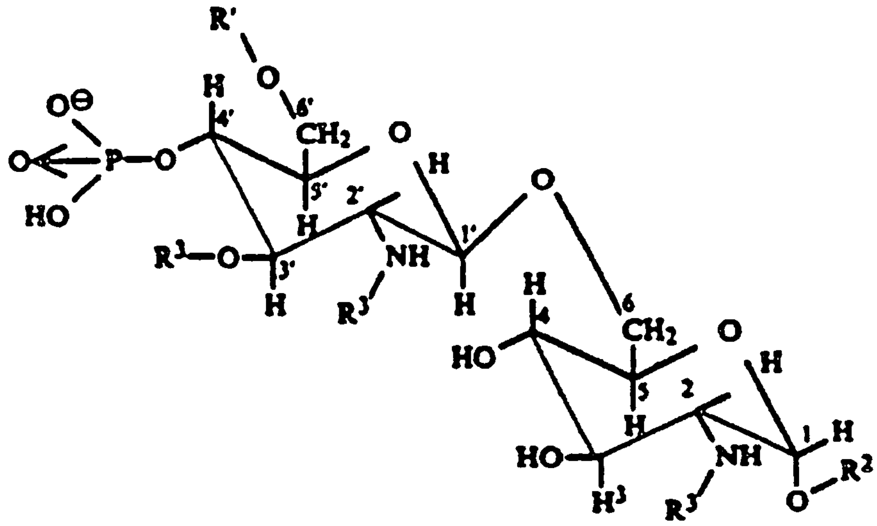

wherein R2 may be H or PO3H2; R3 may be an acyl chain or β-hydroxymyristoyl or a 3-acyloxyacyl residue having the formula:

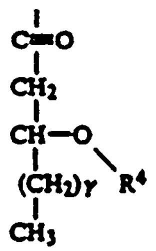

wherein $R^4$ =

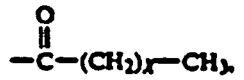

and wherein X and Y have a value of from 0 up to about 20.

Combinations of 3D-MPL and saponin adjuvants derived from the bark of Quillaja Saponaria molina have been described in EP 0 761 231B. WO 95/17210 discloses an adjuvant emulsion system based on squalene, α-tocopherol, and polyoxyethylene sorbitan monooleate (TWEEN80), formulated with the immunostimulant QS21, optionally with 3D-MPL.

Saponins are known as adjuvants in vaccines for systemic administration. The adjuvant and haemolytic activity of individual saponins has been extensively studied in the art (Lacaille-Dubois and Wagner, *supra*). For example, Quil A (derived from the bark of the South American tree Quillaja Saponaria Molina), and fractions thereof, are described in US 5,057,540 and "Saponins as vaccine adjuvants", Kensil, C. R., Crit Rev Ther Drug Carrier Syst, 1996, 12 (1-2):1-55; and EP 0 362 279 B1.

Particulate structures, termed Immune Stimulating Complexes (ISCOMS), comprising fractions of Quil A are haemolytic and have been used in the manufacture of vaccines (Morein, B., EP 0 109 942 B1). These structures have been reported to have adjuvant activity (EP 0 109 942 B1; WO 96/11711).

The haemolytic saponins QS21 and QS 17 (HPLC purified fractions of Quil A) have been described as potent systemic adjuvants, and the method of their production is disclosed in US Patent No.5,057,540 and EP 0 362 279 B 1. Also described in these references is the use of QS7 (a non-haemolytic fraction of Quil-A) which acts as a potent adjuvant for systemic vaccines. Use of QS21 is further described in Kensil et al. (1991. J. Immunology vol 146, 431-437). Combinations of QS21 and polysorbate or cyclodextrin are also known (WO 99/10008). Particulate adjuvant systems comprising fractions of QuilA, such as QS21 and QS7 are described in WO 96/33739 and WO 96/11711.

Other saponins which have been used in systemic vaccination studies include those derived from other plant species such as Gypsophila and Saponaria (Bomford et al., Vaccine, 10(9):572-577, 1992).

Saponins are also known to have been used in mucosally applied vaccine studies, which have met with variable success in the induction of immune responses. Quil-A saponin has previously been shown to have no effect on the induction of an immune response when antigen is administered intranasally (Gizurarson et al. 1994. Vaccine Research 3, 23-29). Whilst, other authors have used this adjuvant with success (Maharaj et al., Can.J.Microbiol, 1986, 32(5): 414-20; Chavali and Campbell, Immunobiology, 174(3):347-59). ISCOMs comprising Quil A saponin have been used in intragastric and intranasal vaccine formulations and exhibited adjuvant activity (McI Mowat et al., 1991, Immunology, 72, 317-322; McI Mowat and Donachie, Immunology Today, 12, 383-385).

QS21, the non-toxic fraction of Quil A, has also been described as an oral or intranasal adjuvant (Sumino et al., J. Virol., 1998, 72(6):4931-9; WO 98/56415).

Saponins are taught in: Lacaille-Dubois, M and Wagner H. (1996. A review of the biological and pharmacological activities of saponins. Phytomedicine vol 2 pp 363-386). Saponins are steroid or triterpene glycosides widely distributed in the plant and marine animal kingdoms. Saponins are noted for forming colloidal solutions in water which foam on shaking, and for precipitating cholesterol. When saponins are near cell membranes they create pore-like structures in the membrane which cause the membrane to burst. Haemolysis of erythrocytes is an example of this phenomenon, which is a property of certain, but not all, saponins.

The present invention relates to the surprising finding that combinations of immunostimulatory oligonucleotides

(CpG), saponin and a lipopolysaccharide are extremely potent adjuvants. Accordingly, there is provided a vaccine combination comprising a combination of saponin and an immunostimulatory oligonucleotide and a lipopolysaccharide with a cancer antigen or derivative thereof as defined in claim 1. In a preferred embodiment the adjuvant formulation comprises a saponin, preferably QS21, an immunostimulatory oligonucleotide, and a 3D-MPL.

Preferably, the vaccine of the present invention may further comprise a carrier. In a preferred form of the present invention the oligonucleotides in the adjuvant and vaccine compositions act synergistically with the combined saponin/ lipolysaccharide in the induction of antigen specific immune responses leading to enhanced tumour regression. The formulations are potent in the induction of immune responses conventionally associated with the Th1-type immune system. Accordingly, the adjuvant combinations are not only suitable for immunoprophylaxis of diseases, but also for immunotherapy of diseases such as cancer.

The formulations contain an anti-tumour antigen and are useful for the immunotherapeutic treatment of cancers. For example, the adjuvant formulation finds utility with tumour rejection antigens such as those for prostrate, breast, colorectal, lung, pancreatic, renal or melanoma cancers. Exemplary antigens include MAGE 1 , 3 and MAGE 4 or other MAGE antigens such as disclosed in WO99/40188, PRAME, BAGE, Lage (also known as NY Eos 1) SAGE and HAGE (WO 99/53061) or GAGE (Robbins and Kawakami, 1996, Current Opinions in Immunology 8, pps 628-636; Van den Eynde et al., International Journal of Clinical & Laboratory Research (submitted 1997); Correale et al. (1997), Journal of the National Cancer Institute 89, p293. Indeed these antigens are expressed in a wide range of tumour types such as melanoma, lung carcinoma, sarcoma and bladder carcinoma.

MAGE antigens for use in the present invention are expressed as a fusion protein with a heterologous fusion partner, being an expression enhancer or an Immunological fusion partner In one embodiment of the present invention, the derivative is a fusion proteins comprising an antigen from the MAGE protein family linked to a heterologous partner preferably MAGE 3. The proteins are expressed as recombinant fusion proteins allowing increased levels to be produced in an expression system as compared to non-fused protein. Thus the fusion partner may assist in providing T helper epitopes(immunological fusion partner), preferably T helper epitopes recognised by humans, or assist in expressing the protein (expression enhancer) at higher yields than the native recombinant protein. Preferably the fusion partner will be both an immunological fusion partner and expression enhancing partner.

In a preferred form of the invention, the immunological fusion partner is derived from protein D, a surface protein of the gram-negative bacterium, Haemophilus influenza B (WO91/18926). Preferably the protein D derivative comprises approximately the first 1/3 of the protein, in particular approximately the first N-terminal 100-110 amino acids. Preferably the protein D derivative is lipidated. Preferably the first 109 residues of the Lipoprotein D fusion partner is included on the N-terminus to provide the vaccine candidate antigen with additional exogenous T-cell epitopes and increase expression level in E-coli (thus acting also as an expression enhancer). The lipid tail ensures optimal presentation of the antigen to antigen presenting cells.

Other fusion partners include the non-structural protein from influenzae virus, NS 1 (hemagglutinin). Typically the N terminal 81 amino acids are utilised, although different fragments may be used provided they include T-helper epitopes.

In another embodiment the immunological fusion partner is the protein known as LYTA. Preferably the C terminal portion of the molecule is used. Lyta is derived from Streptococcus pneumoniae which synthesize an N-acetyl-L-alanine amidase, amidase LYTA, (coded by the lytA gene {Gene, 43 (1986) page 265-272} an autolysin that specifically degrades certain bonds in the peptidoglycan backbone. The C-terminal domain of the LYTA protein is responsible for the affinity to the choline or to some choline analogues such as DEAE. This property has been exploited for the development of E.coli C-LYTA expressing plasmids useful for expression of fusion proteins. Purification of hybrid proteins containing the C-LYTA fragment at its amino terminus has been described {Biotechnology: 10, (1992) page 795-798}. As used herein a preferred embodiment utilises the repeat portion of the Lyta molecule found in the C terminal end starting at residue 178. A particularly preferred form incorporates residues 188 - 305.

The immunological fusion partners noted above are also advantageous in aiding expression. In particular, such fusions are expressed at higher yields than native recombinant MAGE proteins. Such constructs are disclosed in Wo99/40188.

A preferred prostate antigen is known as P501S, sequence ID no 113 of WO98/37814. Immunogenic fragments and portions thereof comprising at least 20, preferably 50, more preferably 100 contiguous amino acids as disclosed in the above referenced patent application. See for example PS 108 (WO 98/50567).

Another tumour associated antigens useful in the context of the present invention is Cripto (Salomon et al Bioessays 199, 21 61 -70, US patent 5654140).

The present invention is also useful in combination with the breast cancer antigen Her 2 neu, Her 2 neu antigens are disclosed inter alia, in US patent 5,801,005. The Her 2 neu must be devoid of a substantial portion of the transmembrane domain and may comprise the entire extracellular domain (comprising approximately amino acid 1 -645) or fragmants thereof and at least an immunogenic portion of or the entire intracellular domain approximately (the C terminal 580 amino acids). In particular, the intracellular portion should comprise the phosphorylation domain or fragments thereof.

Such constructs are disclosed in WO 00/44899. A particularly preferred construct is known as ECD PD a second is known as ECD ΔPD See WO 00/44899.

The Her 2 neu as used herein can be derived from rat, mouse or human.

The Her 2 neu antigen may be the entire Her 2 neu antigen devoid of a functional transmembrane domain or portions thereof. Preferred portions comprises the extracellular domain. In a more preferred embodiment there is provided an fusion protein comprising an extracellular domain linked to a portion of the intracellular domain as disclosed in WO 00/44899.

The present invention is directed to formulations capable of modulating, preferably eliciting or enhancing, immunity to the protein product of Her 2 neu oncogene expression, including for malignancies in a warm-blooded animal where an amplified Her 2 neu gene with a malignancy does not require that the protein expression product of the gene be present on the tumour. For example, overexpression of the gene may be involved with initiation and early stages of tumour formation, but the protein expression may subsequently be reduced or absent. The present invention may be used to elicit or enhance an effective immune response to convert a Her 2 neu positive tumour to Her 2 neu negative, in addition to preventing the establishment of Her 2 neu positive tumours and provoking the regression of existing Her 2 neu positive tumours.

The following abbreviations are used throughout the specification: "ECD" refers to the extracellular domain, "ICD" refers to the intracellular domain, "PD" refers to the phosphorylation domain (ie, the domain that is phosphorylated) that is within the intracellular domain, "ΔPD" refers to a fragment of the phosphorylation domain that is within the phosphorylation domain, and "KD" refers to the kinase domain that is within the intracellular domain. The product of expression of the Her 2 neu gene is referred to herein as the "Her 2 neu protein," also known and referred to as "p 185" or "c-erbB2".

The "Her 2 neu ECD-ICD fusion protein," also referred to herein as "ECD-ICD" or "ECD-ICD fusion protein," refers to a fusion protein (or fragments thereof) comprising the extracellular domain (or fragments thereof) and the intracellular domain (or fragments thereof) of the Her 2 neu protein. These represent preferred antigens to utilise in the context of the present invention. As used herein, the ECD-ICD fusion protein does not include a substantial portion of the Her 2 neu transmembrane domain, and preferably does not include any of the Her 2 neu transmembrane domain.

The terms "Her 2 neu ECD-ICD fusion protein" and "Her 2 neu ECD-PD fusion protein" and their related terms are also understood to refer to fragments thereof, homologs therefore and functional equivalents thereof (collectively referred to as "variants"), such as those in which one or more amino acids which, in preferred embodiments of the invention, either (i) increase the elicitation or enhancement of an immune response as compared to the Her 2 neu protein, or (ii) do not substantially affect elicitation or enhancement of an immune response as compared to the Her 2 neu protein (eg variant stimulates a response by helper T cells or cytotoxic T cells or stimulates the production of antibodies). Specific, non-limiting, examples of variants including exemplary fragments, homologs and functional equivalents of the Her 2 neu ECD-ICD fusion protein and Her 2 neu ECD-PD fusion protein are described in more detail herein. Variants can be "substantially identical" or "substantially similar" to a fusion protein comprising native polypeptide components, and retain the ability to stimulate an immune response.

The Her 2 neu PD is 268 amino acids in length, is intracellular, and can be phosphorylated by protein tyrosine kinases. The region shares no identity with the corresponding part of other tyrosine kinase receptors. Thus, the specificity and uniqueness of this domain makes it particularly preferred for use as a tumour vaccine. However, the expression of this domain alone in bacterial and mammalian cells is problematic. For example, the resultant PD protein is very labile and is not appropriate for large scale production. In one embodiment, this invention thus preferably utilises a fusion comprising all or part of the intracellular domain or the phosphorylation domain to all or part of the Her 2 neu extracellular domain. The ECD-ICD fusion proteins and the ECD-PD fusion proteins are soluble, are secreted and are stable in culture media.

The vaccines of the invention will be useful against any cancer characterised by tumour associated expression of the image, P501S, CRIPTO and Her 2 neu expression. In addition to allowing increased expression of the intracellular domain or phosphorylation domain, or variants thereof, as a fusion protein with the extracellular domain or its variants, the ECD-ICD and ECD-PD fusion proteins provide for an improved vaccine formulation.

Accordingly the present invention provides a vaccine formulation comprising an adjuvant composition, said adjuvant comprising a lipopolysaccharide, as defined in claim 1, a saponin and a immunostimulatory oligonucleotide and a Her 2 neu antigen devoid of its transmembrane domain. The Her 2 neu molecule, may be rat mouse human or a hybrid therof. Preferably the her 2 molecule comprises substantially all of the extracellular domain. By substantially all it is meant no more than 100 amino acids are deleted from the extracellular domain, preferably less than 75, more preferably less than 50 amino acids. It is prefered that the entire extracellular domain be present. The extracellular domain in human Her 2 neu construct of the present invention, comprises preferably the substantially all the N terminal 600 amino acids, more preferably the N terminal 630 amino acids, more preferably about 650 amino acids. The human ICD runs from amino acid 676 to Val 1255. The phosphorylation domain is located in the N terminal portion of the ICD. It is preferred that the constructs utilised in the present invention comprise the phosphorylation domain, but do not include a functional transmembrane domain. Preferably the transmembrane domain is deleted altogether.

Constructs that are particularly suitable for use in the present invention are disclosed in WO/0044899.

It is a preferred embodiment that the Her 2 neu antigen is formulated with 3D-MPL, QS21 and CpG Oligonucleotide together with a liposome or oil in water emulsion carrier. Such formulations produce both a humoral and cellular mediated response. In comparisons with adjuvant formulation comprising just QS21 and 3D-MPL, the formulation of the invention adduced, in mice, advantageously a stronger TH 1 response. CpG only formulations did not produce a significant cell mediated immune response.

The formulations may contain antigens associated with tumour-support mechanisms (e.g. angiogenesis, tumour invasion) for example tie 2 and VEGF.

The preferred oligonucleotides for use in adjuvants or vaccines of the present invention preferably contain two or more dinucleotide CpG motifs separated by at least three, more preferably at least six or more nucleotides. The oligonucleotides are typically deoxynucleotides. In a preferred embodiment the internucleotide in the oligonucleotide is phosphorodithioate, or more preferably a phosphorothioate bond, although phosphodiester and other internucleotide bonds are within the scope of the invention including oligonucleotides with mixed internucleotide linkages. Methods for producing phosphorothioate oligonucleotides or phosphorodithioate are described in US5,666,153, US5,278,302 and WO95/26204.

Examples of preferred oligonucleotides have the following sequences. The sequences preferably contain phosphorothioate modified internucleotide linkages.

OLIGO 1(SEQ ID NO:1): TCC ATG ACG TTC CTG ACG TT (CpG 1826)
OLIGO 2 (SEQ ID NO:2): TCT CCC AGC GTG CGC CAT (CpG 1758)
OLIGO 3(SEQ ID NO:3): ACC GAT GAC GTC GCC GGT GAC GGC ACC ACG
OLIGO 4 (SEQ ID NO:4): TCG TCG TTT TGT CGT TTT GTC GTT (CpG 2006)
OLIGO 5 (SEQ ID NO:5): TCC ATG ACG TTC CTG ATG CT (CpG 1668)

Alternative CpG oligonucleotides may comprise the preferred sequences above in that they have inconsequential deletions or additions thereto.

The CpG oligonucleotides utilised in the present invention may be synthesized by any method known in the art (eg EP 468520). Conveniently, such oligonucleotides may be synthesized utilising an automated synthesizer. They are typically between 10-50 bases in length.

The oligonucleotides utilised in the present invention are typically deoxynucleotides. In a preferred embodiment the internucleotide bond in the oligonucleotide is phosphorodithioate, or more preferably phosphorothioate bond, although phosphodiesters are within the scope of the present invention. Oligonucleotide comprising different internucleotide linkages are contemplated, e.g. mixed phosphorothioate phophodiesters. Other internucleotide bonds which stabilise the oligonucleotide may be used.

The saponins which may be used in the adjuvant combinations of the present invention include those derived from the bark of Quillaja Saponaria Molina, termed Quil A, and fractions thereof, described in US 5,057,540 and "Saponins as vaccine adjuvants", Kensil, C. R., Crit Rev Ther Drug Carrier Syst, 1996, 12 (1-2):1-55; and EP 0 362 279 B1. Particularly preferred fractions of Quil A are QS21, QS7, and QS17.

β-Escin is another preferred haemolytic saponins for use in the adjuvant compositions of the present invention. Escin is described in the Merck index (12$^{th}$ ed: entry 3737) as a mixture of saponins occurring in the seed of the horse chestnut tree, Lat: *Aesculus hippocastanum.* Its isolation is described by chromatography and purification (Fiedler, Arzneimittel-Forsch. 4, 213 (1953)), and by ion-exchange resins (Erbring et al., US 3,238,190). Fractions of escin, □ and □, have been purified and shown to be biologically active (Yoshikawa M, et al. (Chem Pharm Bull (Tokyo) 1996 Aug;44(8):1454-1464)). β-escin is also known as aescin.

Another preferred haemolytic saponin for use in the present invention is Digitonin. Digitonin is described in the Merck index (12$^{th}$ Edition, entry 3204) as a saponin, being derived from the seeds of *Digitalis purpurea* and purified according to the procedure described Gisvold et al., J.Am.Pharm.,Assoc., 1934, 23, 664; and Ruhenstroth-Bauer, Physiol.Chem., 1955, 301, 621. Its use is described as being a clinical reagent for cholesterol determination.

The adjuvant combinations of the present invention may further comprise a carrier, such that the saponin or CpG, or lipolysaccaharide may be associated with a particulate carrier entity to enhance the adjuvanticity of the combination. Particularly preferred systemic vaccines, for example, comprise a carrier molecule.

The CpG used in the adjuvant combinations of the present invention may be in free solution or may be complexed to particulate carriers such as mineral salts (for example, but not restricted to, aluminium or calcium salts), liposomes, ISCOMs, emulsions (oil in water, water in oil, water in oil in water), polymers (such as, but not restricted to polylactic, polyglycolic, polyphosphazine, polyaminoacid, alginate, chitosan) or microparticles. Preferably said carriers are cationic. The vaccines of the present invention further comprise an antigen which may be associated with the CpG-carrier complex, or may not be associated with the CpG-carrier complex. In this case, the antigen may be free suspension or associated with a separate carrier.

The saponins forming part of the composition of the present invention may be separate in the form of micelles, or may be in the form of large ordered structures such as ISCOMs (EP 0 109 942 B1) or liposomes (WO 96/33739)

when formulated with cholesterol and lipid, or in the form of an oil in water emulsion (WO 95/17210). The saponins may preferably be associated with a metallic salt, such as aluminium hydroxide or aluminium phosphate (WO 98/15287). Alternatively the saponin may be associated with a particulate carrier such as chitosan. The saponin may also be in a dry state such as a powder. The final formulations in the form as they are administered to the mucosal surface of the vaccinee are preferably haemolytic in nature. The saponin may or may not be associated physically with the antigen either through direct linkage or by co-interaction with the same particulate carrier molecule (GB9822712.7; WO 98/16247).

The CpG and saponin and lipopolysaccharide in the adjuvants or vaccines of the present invention may themselves be separate or associated. For example, the CpG and saponin may be in free suspension or may be associated via a carrier, more preferably a particulate carrier such as aluminium hydroxide or by a cationic liposome or ISCOM.

A preferred adjuvant combination according to the present invention is composed of one or more CpG oligonucleotides containing at least 3, preferably at least 6 nucleotides between two adjacent CG motifs, together with QS21 and a particulate carrier selected from the group comprising an oil-in-water emulsion or DQ. It is preferrred that the lipopolysacchharide is a di or monophosphoryl lipid derivative, preferably 3 de-O acylated, in particular 3 de O acylated monophosphoryl Lipid A. Most preferably, the adjuvant combination comprises CpG 2006 (SEQ ID NO: 4), or CpG 1758 (SEQ ID NO: 2) or CpG 1826 (SEQ ID NO: 1) mixed with QS21, and a particulate carrier selected from the group comprising an oil-in-water emulsion or DQ. Accordingly, particularly preferred vaccines, for example, comprise such adjuvant combinations and an antigen as defined in claim 1. The preferred vaccine of the present invention is used to generate systemic immune responses after administration to an individual through the systemic route.

The immunogenic combinations of the present invention can comprise an oil based emulsion. Oil emulsion adjuvants have been known for many years, including work on Freunds complete and incomplete mineral oil emulsion adjuvants. Since that time much work has been performed to design stable and well tolerated alternatives to these potent, but reactogenic, adjuvant formulations.

Many single or multiphase emulsion systems have been described. Oil in water emulsion adjuvants per se have been suggested to be useful as adjuvant compositions (EP 0 399 843B), also combinations of oil in water emulsions and other active agents have been described as adjuvants for vaccines (WO 95/17210; WO 98/56414; WO 99/12565; WO 99/11241). Other oil emulsion adjuvants have been described, such as water in oil emulsions (US 5,422,109; EP 0 480 982 B2) and water in oil in water emulsions (US 5,424,067; EP 0 480 981 B).

The oil emulsion adjuvants for use in the present invention may be natural or synthetic, and may be mineral or organic. Examples of mineral and organic oils will be readily apparent to the man skilled in the art.

In order for any oil in water composition to be suitable for human administration, the oil phase of the emulsion system preferably comprises a metabolisable oil. The meaning of the term metabolisable oil is well known in the art. Metabolisable can be defined as "being capable of being transformed by metabolism" (Dorland's Illustrated Medical Dictionary, W.B. Sanders Company, 25th edition (1974)). The oil may be any vegetable oil, fish oil, animal oil or synthetic oil, which is not toxic to the recipient and is capable of being transformed by metabolism. Nuts (such as peanut oil), seeds, and grains are common sources of vegetable oils. Synthetic oils are also part of this invention and can include commercially available oils such as NEOBEE® and others. Squalene (2,6,10,15,19,23-Hexamethyl-2,6,10,14,18,22-tetracosahexaene) is an unsaturated oil which is found in large quantities in shark-liver oil, and in lower quantities in olive oil, wheat germ oil, rice bran oil, and yeast, and is a particularly preferred oil for use in this invention. Squalene is a metabolisable oil virtue of the fact that it is an intermediate in the biosynthesis of cholesterol (Merck index, 10th Edition, entry no.8619).

Particularly preferred oil emulsions are oil in water emulsions, and in particular squalene in water emulsions.

In addition, the most preferred oil emulsion adjuvants of the present invention comprise an antioxidant, which is preferably the oil $\alpha$-tocopherol (vitamin E, EP 0 382 271 B1).

WO 95/17210 and WO 99/11241 disclose emulsion adjuvants based on squalene, $\alpha$-tocopherol, and TWEEN 80, optionally formulated with the immunostimulants QS21 and/or 3D-MPL. WO 99/12565 discloses an improvement to these squalene emulsions with the addition of a sterol into the oil phase. Additionally, a triglyceride, such as tricaprylin (C27H50O6), may be added to the oil phase in order to stabilise the emulsion (WO 98/56414).

The size of the oil droplets found within the stable oil in water emulsion are preferably less than 1 $\mu$m, may be in the range of substantially 30-600nm, preferably substantially around 30-500nm in diameter, and most preferably substantially 150-500nm in diameter, and in particular about 150 nm in diameter as measured by photon correlation spectroscopy. In this regard, 80% of the oil droplets by number should be within the preferred ranges, more preferably more than 90% and most preferably more than 95% of the oil droplets by number are within the defined size ranges. The amounts of the components present in the oil emulsions of the present invention are conventionally in the range of from 2 to 10% oil, such as squalene; and when present, from 2 to 10% alpha tocopherol; and from 0.3 to 3% surfactant, such as polyoxyethylene sorbitan monooleate. Preferably the ratio of oil: alpha tocopherol is equal or less than 1 as this provides a more stable emulsion. Span 85 may also be present at a level of about 1%. In some cases it may be advantageous that the vaccines of the present invention will further contain a stabiliser.

The method of producing oil in water emulsions is well known to the man skilled in the art. Commonly, the

method comprises the mixing the oil phase with a surfactant such as a PBS/TWEEN80™ solution, followed by homogenisation using a homogenizer, it would be clear to a man skilled in the art that a method comprising passing the mixture twice through a syringe needle would be suitable for homogenising small volumes of liquid. Equally, the emulsification process in microfluidiser (M110S microfluidics machine, maximum of 50 passes, for a period of 2 minutes at maximum pressure imput of 6 bar (output pressure of about 850 bar)) could be adapted by the man skilled in the art to produce smaller or larger volumes of emulsion. This adaptation could be achieved by routine experimentation comprising the measurement of the resultant emulsion until a preparation was achieved with oil droplets of the required diameter.

The immunogenic combinations of the present invention may be used as both systemic or mucosal adjuvant. In a particular form of the invention there is provided a systemic vaccine to be administered through the systemic or parenteral route such as intramuscular, intradermal, transdermal, subcutaneous, intraperitoneal or intravenous administration. A preferred route of administration is via the transdermal route, for example by skin patches.

The systemic vaccine preparations of the present invention may be used to protect or treat a mammal susceptible to, or suffering from disease, by means of administering said vaccine by intramuscular, intraperitoneal, intradermal, transdermal, intravenous, or subcutaneous administration. Methods of systemic administration of the vaccine preparations may include conventional syringes and needles, or devices designed for ballistic delivery of solid vaccines (WO 99/27961), or needleless pressure liquid jet device (US 4,596,556; US 5,993,412), or transdermal patches (WO 97/48440; WO 98/28037). The present invention may also be used to enhance the immunogenicity of antigens applied to the skin (transdermal or transcutaneous delivery WO 98/20734 ; WO 98/28037).

Alternatively the vaccine preparations of the present invention may be used to protect or treat a mammal susceptible to, or suffering from disease, by means of administering said vaccine via a mucosal route, such as the oral/alimentary or nasal route. Alternative mucosal routes are intravaginal and intra-rectal. The preferred mucosal route of administration is via the nasal route, termed intranasal vaccination. Methods of intranasal vaccination are well known in the art, including the administration of a droplet, spray, or dry powdered form of the vaccine into the nasopharynx of the individual to be immunised. Nebulised or aerosolised vaccine formulations also form part of this invention. Enteric formulations such as gastro resistant capsules and granules for oral administration, suppositories for rectal or vaginal administration also form part of this invention.

The immunogenic combinations of the present invention, represent a class of mucosal adjuvants suitable for application in humans to replace systemic vaccination by mucosal vaccination.

The adjuvant combinations of the present invention are used in the formulation of vaccines, which vaccines may be administered via the systemic or mucosal route. Preferably, when the vaccines are used for mucosal administration the adjuvant combination comprises a haemolytic saponin.

For mucosal administration preferably the composition of the invention comprise a haemolytic saponin. Haemolytic saponin, or saponin preparation, within the meaning of this invention is to be determined with reference to the following assay.

1. Fresh blood from guinea pigs is washed with phosphate buffered saline (PBS) 3 times in a desk-top centrifuge. After resuspension to the original volume the blood is further diluted 10 fold in PBS.
2. 50 μl of this blood suspension is added to 800 μl of PBS containing two-fold dilutions of surfactant or saponin.
3. After 8 hours the haemolysis is assessed visually or by measuring the optical density of the supernatant. The presence of a red supernatant, which absorbs light at 570 nm indicates the presence of haemolysis.
4. The results are expressed as the concentration of the first saponin dilution at which hemolysis no longer occurs.

For the purposes of this invention the saponin adjuvant preparation is haemolytic if it lyses the erythrocytes at a concentration of less than 0.1%. As means of reference, substantially pure samples of QuilA, QS21, QS7, Digitonin, and β-escin are all haemolytic saponins as defined in this assay. Within the inherent experimental variability of such a biological assay, the saponins of the present invention preferably have a haemolytic activity, of approximately between 0.5-0.00001%, more preferably between 0.05-0.00001%, even more preferably between 0.005-0.00001 %, and most preferably between 0.001-0.0004%. Ideally, said saponins should have a haemolytic activity similar (i.e. within a tenfold difference) to that of QS21.

The vaccines of the present invention may also be administered via the oral route. In such cases the pharmaceutically acceptable excipient may also include alkaline buffers, or enteric capsules or microgranules. The vaccines of the present invention may also be administered by the vaginal route. In such cases, the pharmaceutically acceptable excipients may also include emulsifiers, polymers such as CARBOPOL®, and other known stabilisers of vaginal creams and suppositories. The vaccines of the present invention may also be administered by the rectal route. In such cases the excipients may also include waxes and polymers known in the art for forming rectal suppositories.

Preparations of more than one saponin in the adjuvant combinations of the present invention are also form part of the present invention. For example, combinations of at least two of the following group comprising QS21, QS7, Quil A, β-escin, or digitonin. Additionally, the compositions of the present invention may comprise combinations of more

than one immunostimulatory oligonucleotide.

Alternatively the formulations may be combined with vaccine vehicles composed of chitosan or other polycationic polymers, polylactide and polylactide-co-glycolide particles, poly-N-acetyl glucosamine-based polymer matrix, particles composed of polysaccharides or chemically modified polysaccharides, liposomes and lipid-based particles, particles composed of glycerol monoesters, etc. The saponins may also be formulated in the presence of cholesterol to form particulate structures such as liposomes or ISCOMs. Furthermore, the saponins may be formulated together with a polyoxyethylene ether or ester, in either a non-particulate solution or suspension, or in a particulate structure such as a paucilamelar liposome or ISCOM. The saponins may also be formulated with excipients such as Carbopol$^R$ to increase viscosity, or may be formulated in a dry powder form with a powder excipient such as lactose.

Particularly preferred adjuvants are combinations of 3D-MPL and QS21 (EP 0 671 948 B1), oil in water emulsions comprising 3D-MPL and QS21 (WO 95/17210, WO 98/56414), or 3D-MPL formulated with other carriers (EP 0 689 454 B1) in combination with the CpG oligonucleotides as herein described. The amount of CpG or immunostimulatory oligonucleotides in the adjuvants or vaccines of the present invention is generally small, but depending on the vaccine formulation may be in the region of 1-1000μg per dose, preferably 1-500μg per dose, and more preferably between 1 to 100μg per dose.

The amount of saponin for use in the adjuvants of the present invention may be in the region of 1-1000μg per dose, preferably 1-500μg per dose, more preferably 1-250μg per dose, and most preferably between 1 to 100μg per dose. The ratio of CpG:saponin (w/w) will, therefore, be in the range of 1:1000 to 1000:1, and will typically be in the range of 1:100 to 100:1, and preferably in the range of 1:10 to 1:1 or 1:1 to 10:1, and most preferably 1:1, 4:1 or 10:1.

The formulations of the present invention maybe used for both prophylactic and therapeutic purposes. Accordingly, there is provided the use of a combination of a saponin, a lipopolysaccharide and a CpG molecule together with a cancer antigen, as defined in claim 1 in the manufacture of a vaccine for the prophylaxis and the treatment of cancer, in particular breast and prostate carcinomas. In a further aspect of the present invention there is provided a vaccine or adjuvant combination, comprising a lipopolysaccharide, a saponin and CpG, as herein described for use as a medicament. Vaccine preparation is generally described in New Trends and Developments in Vaccines, edited by Voller et al., University Park Press, Baltimore, Maryland, U.S.A. 1978.

The invention therefore provides a method to prevent an individual from contracting a disease selected from the group comprising prostate, breast, colorectal, lung, pancreatic, renal, ovarian or melanoma cancers; comprising the administration of a composition as substantially described herein through the systemic route of said individual.

Examples of suitable pharmaceutically acceptable excipients for use in the combinations of the present invention include water, phosphate buffered saline, isotonic buffer solutions.

**Example 1:**

- ECD-PD was produced in CHO cells according to the methods of WO 00/44899. The formulations were tested in mice and rabbits.
- Formulations were compared against a number of controls.

**SBAS1+SBAS7:**

ECD-PD formulated with CpG oligonucleotide 2006 3D-MPL, QS21 in liposomes.

**SBAS1 formulation**

Comprising QS21 in liposomes and 3D-MPL associated with the liposomes were prepared according to the procedures of EP 0822831.

**SBAS1+SBAS7 formulation**

To the formulation above CpG oligonucleotide 2006 was added. The antigen was admixed to the adjuvant formulation prior to use.

**SBAS7 + SBAS2-based formulations ( mice)**

For one dose of 50μl of vaccine, the ECD-PD protein (25μg) was diluted in 10 fold concentrated PBS pH 6.8

and H2O before consecutive addition of an oil in water emulsion comprising SB62: which is prepared by and comprises 5% squalene 5% tocopherol 2.0% tween 80; the particle size was 180nm

**Preparation of emulsion SB62 (2 fold concentrate)**

Tween 80 is dissolved in phosphate buffered saline (PBS) to give a 2% solution in the PBS. To provide 100 ml two fold concentrate emulsion 5g of DL alpha tocopherol and 5ml of squalene are vortexed to mix thoroughly. 90ml of PBS/Tween solution is added and mixed thoroughly. The resulting emulsion is then passed through a syringe and finally microfluidised by using an M11OS microfluidics machine. The resulting oil droplets have a size of approximately 180 nm., 3D-MPL (10μg), QS21 (10μg). 50μg CpG ODN 2006 were then added followed 30 minutes later by the addition of 50 μg/ml thiomersal as preservative. All incubations were carried out at room temperature with agitation.
SBAS 2 formulations were prepared as above, but without the addition of the CpG oligonucleotide.
SBAS7 is CpG oligonucleotide 2006

**SBAS7 + SBAS2-based formulations ( Rabbit)**

For one dose of 500μl of vaccine, the ECD-PD protein (100μg) was diluted in 10 fold concentrated PBS pH 6.8 and H2O before consecutive addition of SB62 250μl, 3D-MPL (100μg), QS21 (100μg) and 500μg of CpG ODN 2006 followed 30 minute later, by the addition of 50 μg/ml thiomersal as preservative. All incubations were carried out at room temperature with agitation.

**Example 2: Tumour challenge experiments**

Groups of F1 (C57 x Balb c) mice (8 mice/group) were injected with 1/10 of the human dose of antigen (25μg) at days 0-14-28-42 and challenged at day 56 with TC1 cells expressing Her2 at a close 2 10e6 TC 1 Her2 cell/animal administered subscutaneously.
TC1 cells for ½ the animals spleens were collected at day 56 and the animals bled.
As shown in the figure 1 the addition of a CpG oligonucleotide to a 3D-MPL/QS21 formulation synergistically enhances tumour regression and only these formulations brought about complete tumour regression in the mice.

**Example 3: Immunogenicity of ECD-PD in different adjuvants in rabbits**

6 groups of 4 rabbits were immunised at days 0, 21 and 42 respectively with 100μg of ECD-PD in AS02, AS01, AS05, AS06 (CpG 2006 absorbed on alum), AS07 and AS02B+AS07.
Serology was analysed 14 days post III and table 1 shows that the formulations of the present invention were superior to other formulations tested in raising high titre antibody responses.

**Table 1**

| | pre | 14postIII |
|---|---|---|
| AS02B | 50 | 96923 |
| AS01B | 173 | 196637 |
| AS5 | 144 | 76221 |
| AS6 | 142 | 74180 |
| AS07A | 480 | 3904 |
| AS02B+AS07A | 94 | 362713 |

**Example 4: Immunogenicity of Her 2 neu, ECD-PD in adult Rhesus monkeys**

Adult Rhesus monkeys were immunised with ECD-PD in various adjuvant formulations:

| | |
|---|---|
| AS02 B | - QS21, 3DMPL, in oil water emulsion |
| AS01 | - QS21 3D-MPL in liposome |
| AS05 | - QS21 in liposome |
| AS06 | - CpG 2006 alum |
| AS07 | - CpG 2006 |

(continued)

AS02B+ AS07 - see example 1 for details.

Vaccination illicited a higher antibody response in the formulations of the present invention (AS)2 + AS07). See figure 1.

Further analysis showed the antibody response to be polyclonal and demonstrate an inhibitory activity on the invitro growth of a human breast cancer cell line (SKBR3) over expressing the Iter 2 neu molecule. Herceptin, a monoclonal antibody for the treatment of Her 2 neu expressing tumours is able to inhibit the growth of this cell line.

The antibodies generated after active vaccination with the formulation were thus seen to be functional.

**Example 5: Immunisation of mice with ECD-PD antigen**

This experiment was designed to investigate a range of adjuvant formulations with the antigen which is a fusion of the extracellular domain of Her 2 neu linked to the phosphorylation domain (ECD-PD), which was produced in CHO cells according to the methods of WO 00/44899.

| Group | Antigen (25μg) | Adjuvant |
|---|---|---|
| 1 | ECD-PD | none (Phosphate Buffered Saline (PBS)) |
| 2 | ECD-PD | Liposomes with QS21 and 3D-MPL in membrane |
| 3 | ECD-PD | tocol containing oil in water emulsion with QS21 and 3D-MPL |
| 4 | ECD-PD | CpG |
| 5 | ECD-PD | Liposomes with QS21 and 3D-MPL in membrane + CpG |
| 6 | ECD-PD | tocol containing oil in water emulsion with QS21 and 3D-MPL + CpG |
| 7 | ECD-PD | 3D-MPL + CpG |
| 8 | ECD-PD | QS21 + CpG |
| 9 | ECD-PD | tocol containing oil in water emulsion + CpG |
| 10 | ECD-PD | Liposomes with QS21 in membrane + CpG |
| 11 | ECD-PD | Liposomes with 3D-MPL in membrane + CpG |

The tocol containing oil in water emulsions used in the above groups used D, L, -tocopherol (CAS No. 10191-41-0; chemical name: (2RS,4'RS, 8'RS)-2, 5, 7, 8-tetramethyl-2-(4', 8', 12'-trimethyl-tridecyl)-6-chromanol)); which is commercially available from ROCHET™. If present the tocol was present in an oil in water emulsion comprising 2.5% by volume, in combination with squalene 2.5% by volume. Both oils were mixed, and polyoxyethylene sorbitan monooleate (Tween 80™) was added, prior to microfluidisation (M110S microfluidics machine, maximum of 50 passes, for a period of 2 minutes at maximum pressure imput of 6 bar (output pressure of about 850 bar) as described in WO 95/17210). Accordingly, groups 3, 6, and 9 were based on the above tocol emulsion with the addition of aqueous QS21, 3D-MPL or CpG.

QS21 and 3D-MPL if present in any of the vaccine groups above were included at 5μg/dose; CpG (OLIGO 4 (SEQ ID NO:4): TCG TCG TTT TGT CGT TTT GTC GTT) was added at 50μg dose.

The adjuvants as used for group 2, 5, 10 were prepared according to techniques as described in EP 0 822 831 B1 (the contents of which are incorporated herein by reference). Group 11 comprised 3D-MPL in the membrane of a liposome. Briefly, the 3D-MPL, dioleoyl phosphatydyl choline and cholesterol were mixed together and microfluidised into unilamellar liposomes (as described in EP 0 822 831 B1 - with the omission of QS21).

The adjuvants used in groups 4, 7 and 8 were in aqueous suspension or solution.

**Vaccination procedure**

Groups of B6F1 mice were vaccinated on four occasions (in 50μl volumes), intramuscularly, 14 days apart. 14 days post the 4th vaccine dose, the mice were challenged subcutaneously with 2X106 TC1 tumour cell expressing the Her 2 neu.

The Her 2 neu-TC1 tumour cell lines was produced by transduction of TC1 cells by retroviral vectors coding for Her 2 neu. After a selection period with blastocydin, resistant clones were isolated and screened by FACS for Her 2 neu expression. The clone with the highest Her 2 neu expression was selected, and a challenge dose of 2X106 was identified to have a similar Kinetic of growth as the wild-type TC1 cells and to give rise to a developing tumour in 100% of the control animals.

The size of the individual tumors were measured twice a week and expressed as a group mean.

**Results**

Figure 3 shows the tumour growth results for groups 1, 2, 4, 5 and 6. Figure 4 shows the tumour growth results for groups 1, 5, 6, 7 and 11. Figure 5 shows the tumour growth results for groups 1, 5, 6, 8, 9 and 10. The only vaccines that induced a complete regression of the tumour were vaccine containing both an immunostimulatory oligonucleotide and a saponin.

Figures 6 and 7 show the lymphoproliferation of splenocytes in vitro after incubation with the 5μg/ml of immunogen (ECD-PD) or extracellular domain (ECD) or intracellular domain (ICD) or Her 2 neu.

Figures 8 and 9 show the humoral immune response to the immunogen (ECD-PD) in terms of total Ig as measured by ELISA (FIG. 8) or IgG isotype distribution within these responses (FIG. 9).

**Conclusion:**

Post 3 injections, the antibody induction is

AS02B+AS07A > AS01B > AS02B = AS06 = AS05 > AS07A

**General conclusion**

The adjuvant tested (AS1, AS2, AS7) have similar effect. However,the combination of AS 1 and AS7 or AS2 and AS7 are more effective adjuvants. CMI is clearly shown after 4 vaccinations in animals receiving the combined adjuvant on the whole molecule ECD-PD, but also on each part separately (ECD and ICD). The formulations of the present invention are very effective in inducing tumour regression.

**Example 6: Immunisation of mice with P703P antigen**

This experiment was designed to investigate a range of adjuvant formulations with the antigen which is a fusion of the antigen Prostase (Ferguson, et al. (Proc. Natl. Acad. Sci. USA 1999, 96, 3114-3119)) and the N-terminal 1-81 fragment of NS 1 from the Influenza virus (P703P-NS1).

| Group | Antigen (25μg) | Adjuvant |
|---|---|---|
| 1 | P703P-NS 1 | none (Phosphate Buffered Saline (PBS)) |
| 2 | P703P-NS 1 | CpG |
| 3 | P703P-NS 1 | Liposomes with QS21 in membrane + CpG |
| 4 | P703P-NS 1 | Liposomes with QS21 and 3D-MPL in membrane + CpG |
| 5 | P703P-NS 1 | tocol containing oil in water emulsion with QS21 and 3D-MPL + CpG |
| 6 | P703P-NS 1 | tocol containing oil in water emulsion + CpG |

The tocol containing oil in water emulsions used in the above groups used D, L, α-tocopherol (CAS No. 10191-41-0; chemical name: (2RS,4'RS, 8'RS)-2, 5, 7, 8-tetramethyl-2-(4', 8', 12'-trimethyl-tridecyl)-6-chromanol)); which is commercially available from ROCHE™. If present the tocol was present in an oil in water emulsion comprising 2.5% by volume, in combination with squalene 2.5% by volume. Both oils were mixed, and polyoxyethylene sorbitan monooleate (Tween 80™) was added, prior to microfluidisation (M110S microfluidics machine, maximum of 50 passes, for a period of 2 minutes at maximum pressure input of 6 bar (output pressure of about 850 bar) as described in WO 95/17210). Accordingly, groups 5 and 6 were based on the above tocol emulsion with the addition of aqueous QS21, 3D-MPL and/or CpG.

QS21 and 3D-MPL if present in any of the vaccine groups above were included at 5μg/dose; CpG (OLIGO 4 (SEQ ID NO:4): TCG TCG TTT TGT CGT TTT GTC GTT) was added at 50μg dose.

The adjuvants as used for group 3 and 4 were prepared according to techniques as described in EP 0 822 831 B1 (the contents of which are incorporated herein by reference).

*Vaccination procedure*

Groups of B6F 1 mice were vaccinated on four occasions (in 50μl volumes), intramuscularly, 14 days apart.

*Results*

Figures 10 and 11 show the in vitro lymphoproliferation of splenocytes post second and 14 days post fourth vaccinations, after in vitro incubation with the 3μg/ml of immunogen (NS1-P703P) or pichia expressed P703P (15μg/ml) or a non-specific NS1-OspA fusion protein.

Figures 12 and 13 show the humoral immune response to the immunogen (NS1-P703P) in terms of total Ig as measured by mid-point titre ELISA (FIG. 10) or IgG isotype distribution within these responses (FIG. 11).

## Claims

**1.** An immunogenic composition comprising a cancer antigen selected from the group:

i) an antigen from the MAGE protein family linked to a heterologous fusion partner;
ii) P501S;
iii) Cripto;
iv) Her-2/neu antigen derivative devoid of a substantial portion of the Her-2/neu transmembrane domain,

a lipopolysaccaride selected from the group:

a) Monophosphoryl Lipid A
b) 3-O-Deacylated Monophosphoryl Lipid A
c) Disphosphoryl Lipid A, and

an adjuvant composition comprising a saponin, together with an immunostimulatory oligonucleotide.

**2.** A composition as claimed in claim 1 wherein the saponin is QS21.

**3.** An immunogenic composition as claimed in claim 1 or 2 wherein the immunostimulatory oligonucleotide contains at least two CpG motifs.

**4.** An immunogenic composition as claimed in any of claims 1 to 3 wherein the immunostimulatory oligonucleotide is selected from the group:

SEQ ID No 1 - TCC ATG ACG TTC CTG ACG TT (CpG 1826)
SEQ ID No 2 - TCT CCC AGC GTG CGC CAT (CpG 1758)
SEQ ID No 3 - ACC GAT GAC GTC GCC GGT GAC GGC AAC ACG
SEQ ID No 4 - TCG TCG TTT TGT CGT TTT GTC GTT (CpG 2006)
SEQ ID No 5 - TCC ATG ACG TTC CTG ATG CT (CpG 1668).

**5.** A composition as claimed in any of claims 1 to 4 wherein the saponin is formulated to form ISCOMS or liposomes.

**6.** A composition as claimed in any of claims 1 to 4 wherein the saponin is present in an oil or water emulsion.

**7.** A composition as claimed in any of claims 1 to 6 comprises substantially all of the extracellular domain of Her-2/neu.

**8.** A composition as claimed in claim 7 wherein the Her-2/neu molecule is devoid of a functional transmembrane domain.

9. A composition as claimed in claim 7 or 8 which additionally comprises the phosphorylation domain of Her-2/neu.

10. Use of a combination of a saponin, an immunostimulatory oligonucleotide, an lipopolysaccaride selected from the group:

a) Monophosphoryl Lipid A
b) 3-O-Deacylated Monophosphoryl Lipid A
c) Disphosphoryl Lipid A, and

and a cancer antigen selected from the group:

i) an antigen from the MAGE protein family linked to a heterologous fusion partner;
ii) P501S;
iii) Cripto;
iv) Her-2/neu antigen derivative devoid of a substantial portion of the Her-2/neu transmembrane domain,

in the manufacture of a medicament for the treatment or prophylaxis of tumours.

## Patentansprüche

1. Immunogene Zusammensetzung, die ein Krebsantigen, das aus der folgenden Gruppe ausgewählt ist:

i) ein Antigen aus der MAGE-Proteinfamilie, das an einen heterologen Fusionspartner gebunden ist;
ii) P501S;
iii) Cripto;
iv) Her-2/neu-Antigenderivat ohne einen substantiellen Teil der Her-2/neu-Transmembrandomäne,

ein Lipopolysaccharid, das aus der folgenden Gruppe ausgewählt ist:

a) Monophosphoryl-Lipid A
b) 3-O-desacyliertes Monophosphoryl-Lipid A
c) Disphosphoryl-Lipid A, und

eine Hilfsstoffzusammensetzung umfasst, die ein Saponin zusammen mit einem immunstimulierenden Oligonukleotid umfasst.

2. Zusammensetzung gemäß Anspruch 1, worin das Saponin QS21 ist.

3. Immunogene Zusammensetzung gemäß Anspruch 1 oder 2, worin das immunstimulierende Oligonukleotid mindestens zwei CpG-Motive enthält.

4. Immunogene Zusammensetzung gemäß einem der Ansprüche 1 bis 3, worin das immunstimulierende Oligonukleotid aus der folgenden Gruppe ausgewählt ist:

SEQ. ID. NR. 1 - TCC ATG ACG TTC CTG ACG TT (CpG 1826)
SEQ. ID. NR. 2 - TCT CCC AGC GTG CGC CAT (CpG 1758)
SEQ. ID. NR. 3 - ACC GAT GAC GTC GCC GGT GAC GGC AAC ACG
SEQ. ID. NR. 4 - TCG TCG TTT TGT CGT TTT GTC GTT (CpG 2006)
SEQ. ID. NR. 5 - TCC ATG ACG TTC CTG ATG CT (CpG 1668).

5. Zusammensetzung gemäß einem der Ansprüche 1 bis 4, worin das Saponin formuliert ist, um ISCOMS oder Liposome zu bilden.

6. Zusammensetzung gemäß einem der Ansprüche 1 bis 4, worin das Saponin in einer Öl- oder Wasseremulsion vorliegt.

7. Zusammensetzung gemäß einem der Ansprüche 1 bis 6, die im Wesentlichen alles der extrazellulären Domäne

von Her-2/neu umfasst.

**8.** Zusammensetzung gemäß Anspruch 7, worin das Her-2/neu-Molekül ohne eine funktionelle Transmembrandomäne ist.

**9.** Zusammensetzung gemäß Anspruch 7 oder 8, die zusätzlich die Phosphorylierungsdomäne von Her-2/neu umfasst.

**10.** Verwendung einer Kombination aus einem Saponin, einem immunstimulierenden Oligonukleotid, einem Lipopolysaccharid, das aus der folgenden Gruppe ausgewählt ist:

a) Monophosphoryl-Lipid A
b) 3-O-desacyliertes Monophosphoryl-Lipid A
c) Disphosphoryl-Lipid A, und

einem Krebsantigen, das aus der folgenden Gruppe ausgewählt ist:

i) ein Antigen aus der MAGE-Proteinfamilie, das an einen heterologen Fusionspartner gebunden ist;
ii) P501S;
iii) Cripto;
iv) Her-2/neu-Antigenderivat ohne einen substantiellen Teil der Her-2/neu-Transmembrandomäne,

bei der Herstellung eines Medikaments zur Behandlung oder Prophylaxe von Tumoren.

## Revendications

**1.** Composition immunogène comprenant un antigène associé à un cancer, choisi dans le groupe :

i) un antigène provenant de la famille des protéines MAGE lié à un partenaire de fusion hétérologue ;
ii) P501S ;
iii) Cripto ;
iv) un dérivé d'un antigène Her-2/neu dénué d'une partie conséquente du domaine transmembranaire de Her-2/neu,

un lipopolysaccaride choisi dans le groupe :

a) monophosphoryl lipide A
b) monophosphoryl lipide A 3-O-désacylé
c) disphosphoryl lipide A, et

une composition adjuvante comprenant une saponine, conjointement avec un oligonucléotide immunostimulateur.

**2.** Composition selon la revendication 1, dans laquelle la saponine est QS21.

**3.** Composition immunogène selon la revendication 1 ou 2, dans laquelle l'oligonucléotide immunostimulateur contient au moins deux motifs CpG.

**4.** Composition immunogène selon l'une quelconque des revendications 1 à 3, dans laquelle l'oligonucléotide immunostimulateur est choisi dans le groupe :

SEQ ID NO 1 - TCC ATG ACG TTC CTG ACG TT (CpG 1826)
SEQ ID NO 2 - TCT CCC AGC GTG CGC CAT (CpG 1758)
SEQ ID NO 3 - ACC GAT GAC GTC GCC GGT GAC GGC AAC ACG
SEQ ID NO 4 - TCG TCG TTT TGT CGT TTT GTC GTT (CpG 2006)
SEQ ID NO 5 - TCC ATG ACG TTC CTG ATG CT (CpG 1668).

**5.** Composition selon l'une quelconque des revendications 1 à 4, dans laquelle la saponine est formulée de manière à former des ISCOMs ou des liposomes.

6. Composition selon l'une quelconque des revendications 1 à 4, dans laquelle la saponine est présente dans une émulsion huileuse ou aqueuse.

7. Composition selon l'une quelconque des revendications 1 à 6, comprenant sensiblement la totalité du domaine extracellulaire de Her-2/neu.

8. Composition selon la revendication 7, dans laquelle la molécule de Her-2/neu est dénuée d'un domaine transmembranaire fonctionnel.

9. Composition selon la revendication 7 ou 8, qui comprend de plus le domaine de phosphorylation de Her-2/neu.

10. Utilisation d'une combinaison d'une saponine, d'un oligonucléotide immunostimulateur, d'un lipopolysaccaride choisi dans le groupe :

a) monophosphoryl lipide A
b) monophosphoryl lipide A 3-O-désacylé
c) disphosphoryl lipide A, et

un antigène associé à un cancer choisi dans le groupe :

i) un antigène provenant de la famille des protéines MAGE lié à un partenaire de fusion hétérologue ;
ii) P501S ;
iii) Cripto ;
iv) un dérivé d'antigène Her-2/neu dénué d'une partie conséquente du domaine transmembranaire de Her-2/neu,

dans la fabrication d'un médicament pour le traitement ou la prophylaxie de tumeurs.

FIGURE 1

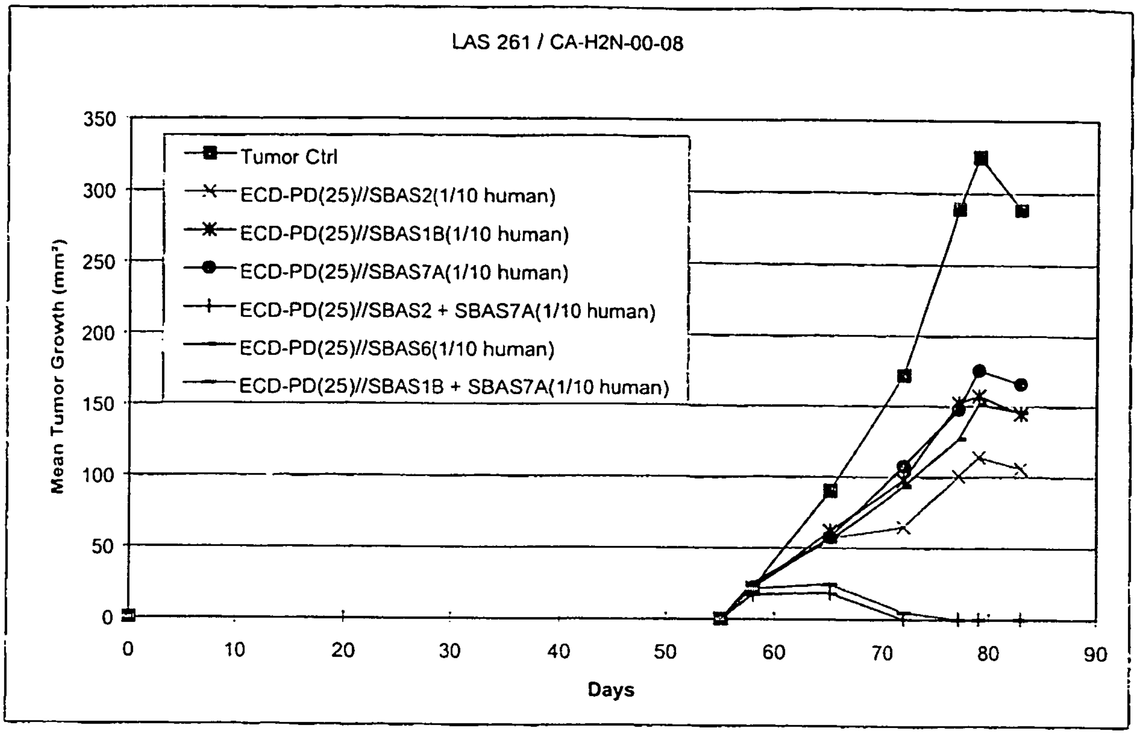
LAS 261 / CA-H2N-00-08
Tumor Ctrl
ECD-PD(25)//SBAS2(1/10 human)
ECD-PD(25)//SBAS1B(1/10 human)
ECD-PD(25)//SBAS7A(1/10 human)
ECD-PD(25)//SBAS2 + SBAS7A(1/10 human)
ECD-PD(25)//SBAS6(1/10 human)
ECD-PD(25)//SBAS1B + SBAS7A(1/10 human)
Mean Tumor Growth (mm²)
350
300
250
200
150
100
50
0
0
10
20
30
40
50
60
70
80
90
Days

FIGURE 2

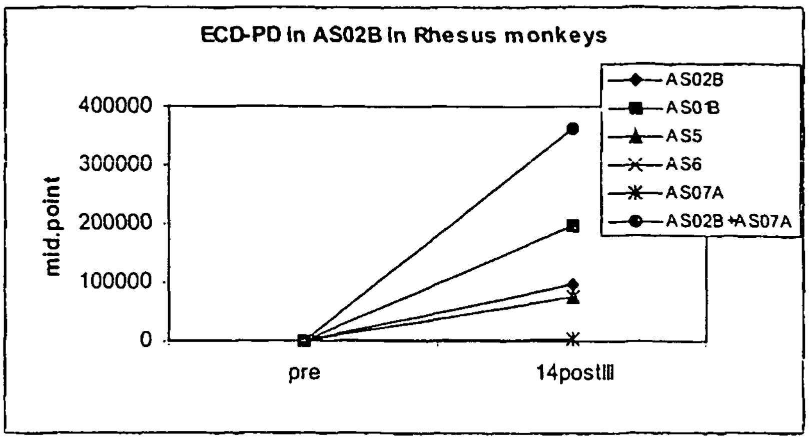
ECD-PD in AS02B in Rhesus monkeys
400000
300000
200000
100000
0
mid.point
pre
14postIII
AS02B
AS01B
AS5
AS6
AS07A
AS02B+AS07A

FIGURE 3 - In vivo tumour growth post vaccination

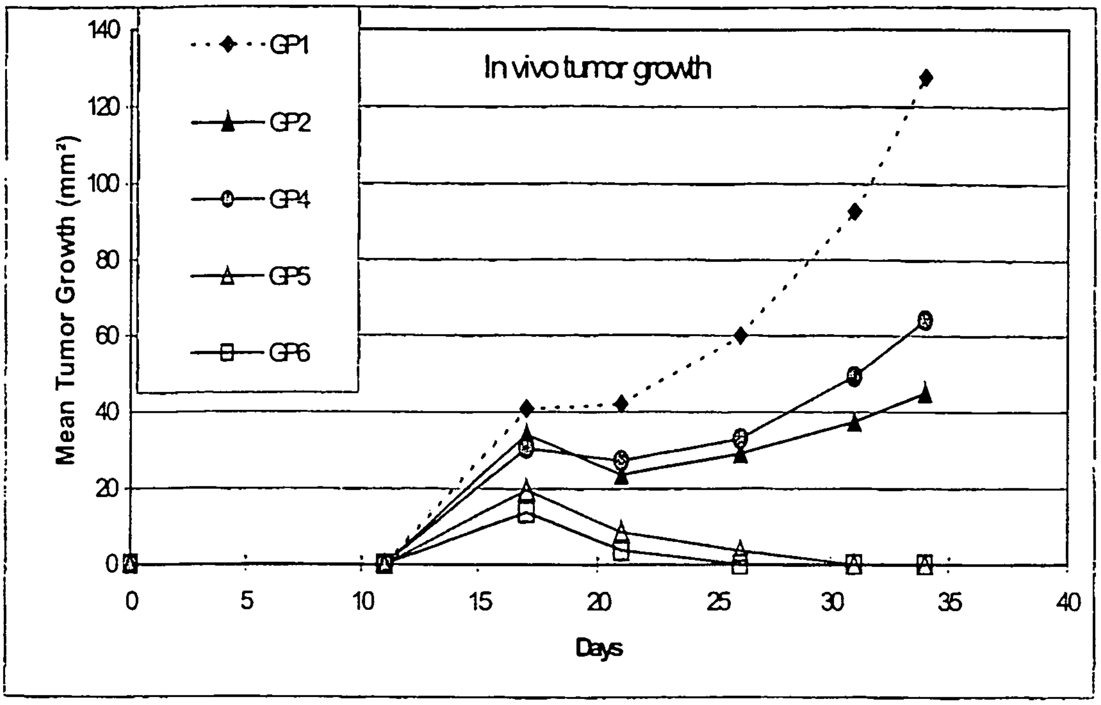

FIGURE 4

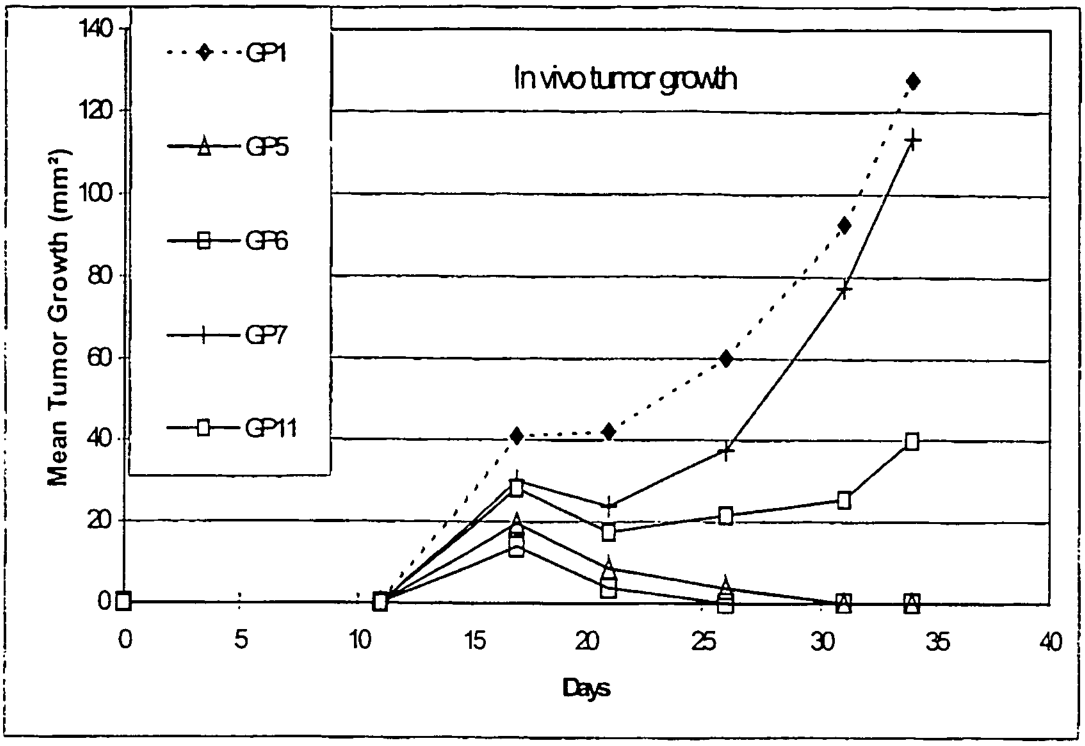
In vivo tumor growth
Mean Tumor Growth (mm²)
Days
GP1
GP5
GP6
GP7
GP11
0
20
40
60
80
100
120
140
0
5
10
15
20
25
30
35
40

FIGURE 5

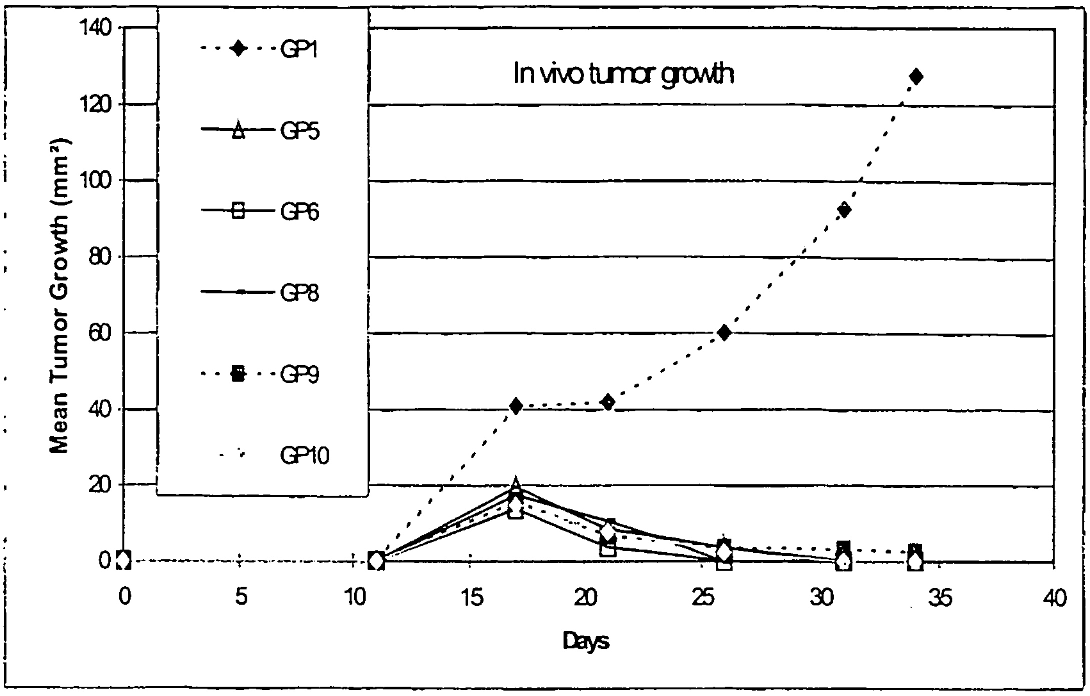
In vivo tumor growth
Mean Tumor Growth (mm²)
Days
GP1
GP5
GP6
GP8
GP9
GP10
0
20
40
60
80
100
120
140
0
5
10
15
20
25
30
35
40

FIGURE 6 - Lymphoproliferation (post vaccination, pre-tumour challenge)

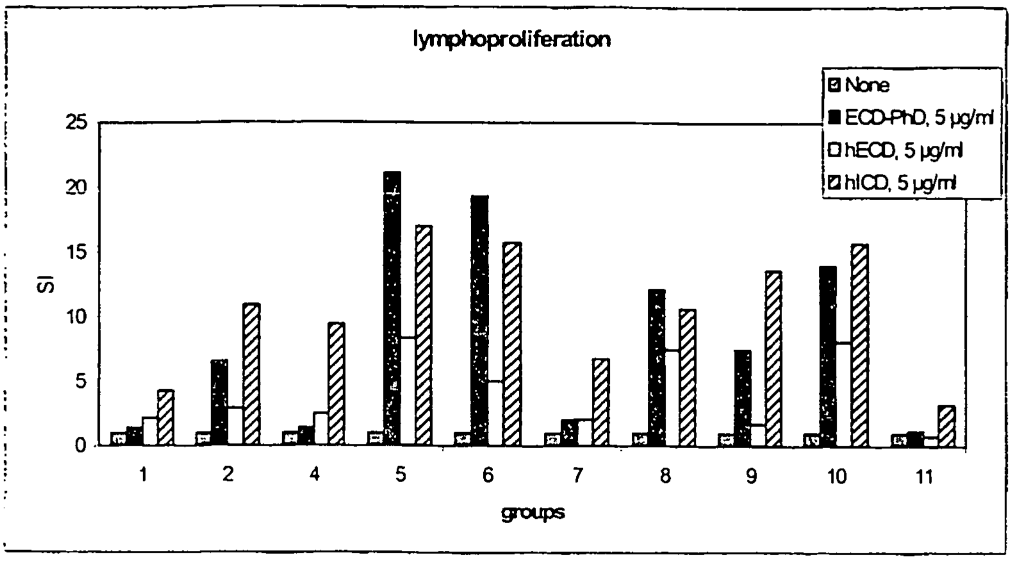

FIGURE 7 - Lymphoproliferation (post tumour challenge)

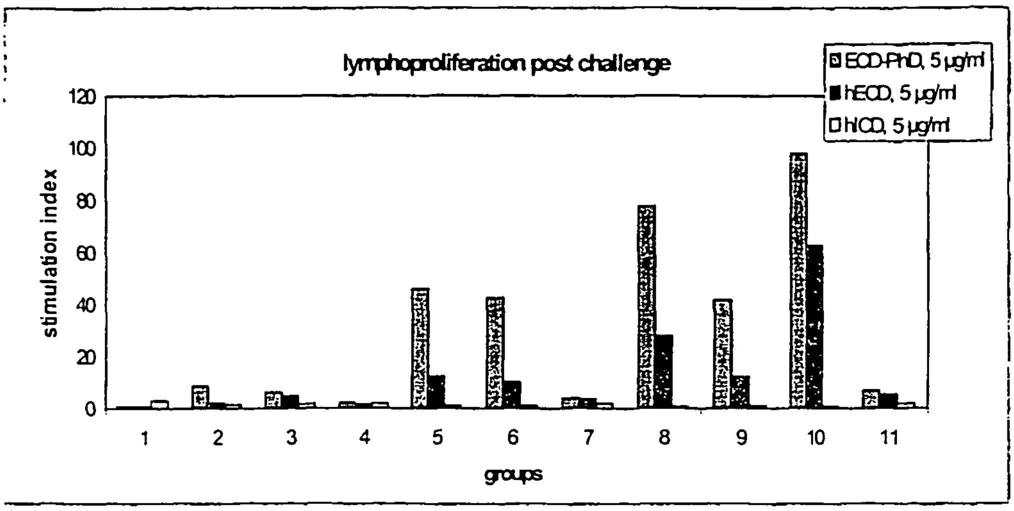

FIGURE 8 - Total anti-ECD Ig response post vaccination.

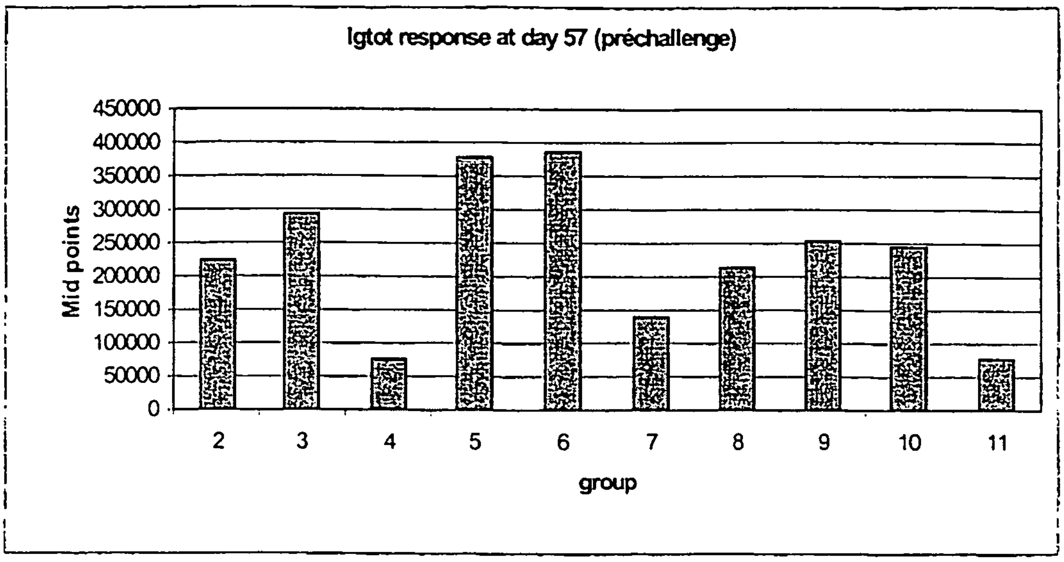

FIGURE 9 - Isotype distribution induced by vaccines

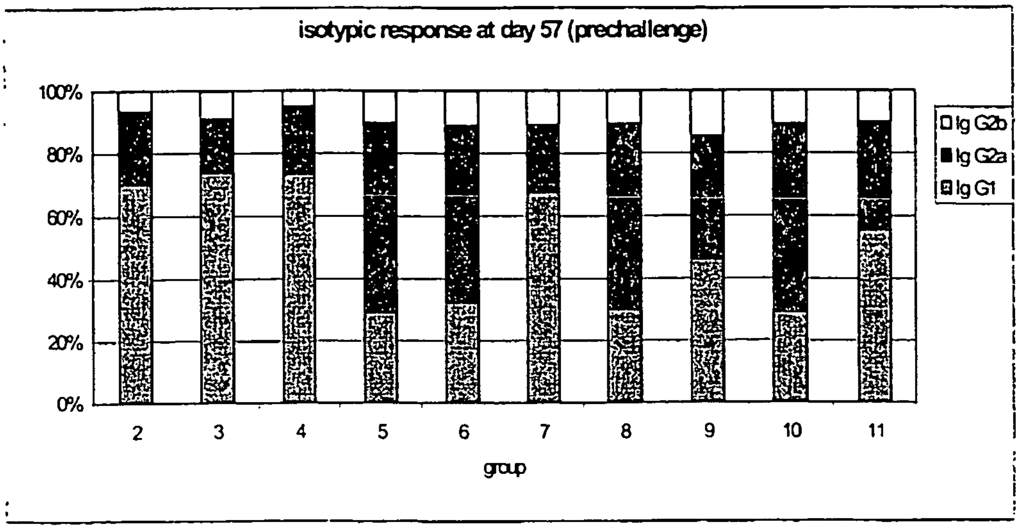

FIGURE 10 - Lymphoproliferation post second P703 vaccination

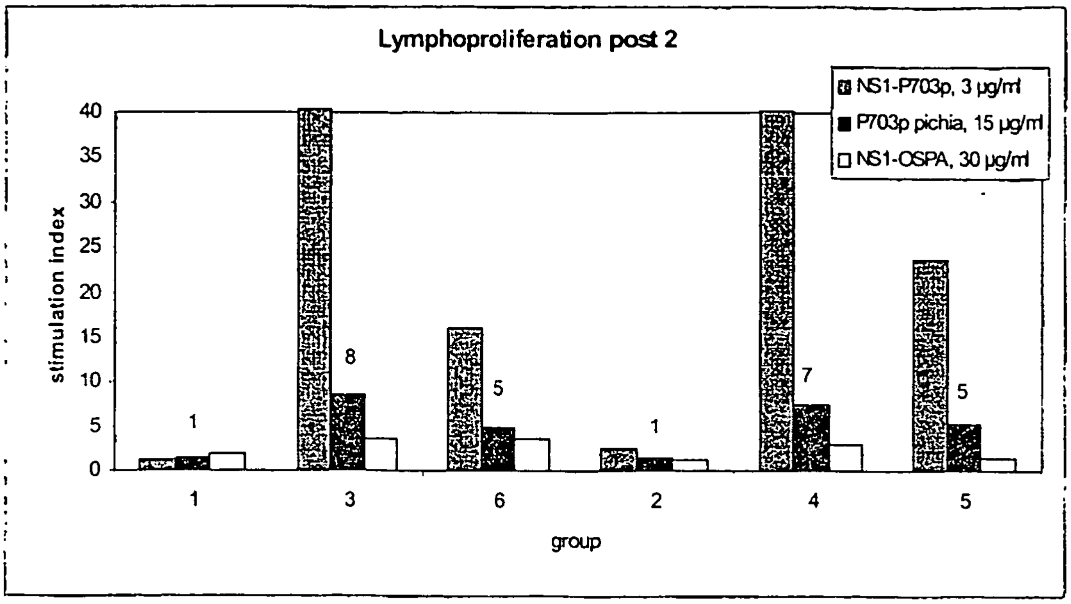

FIGURE 11 - Lymphoproliferation post fourth P703 vaccination

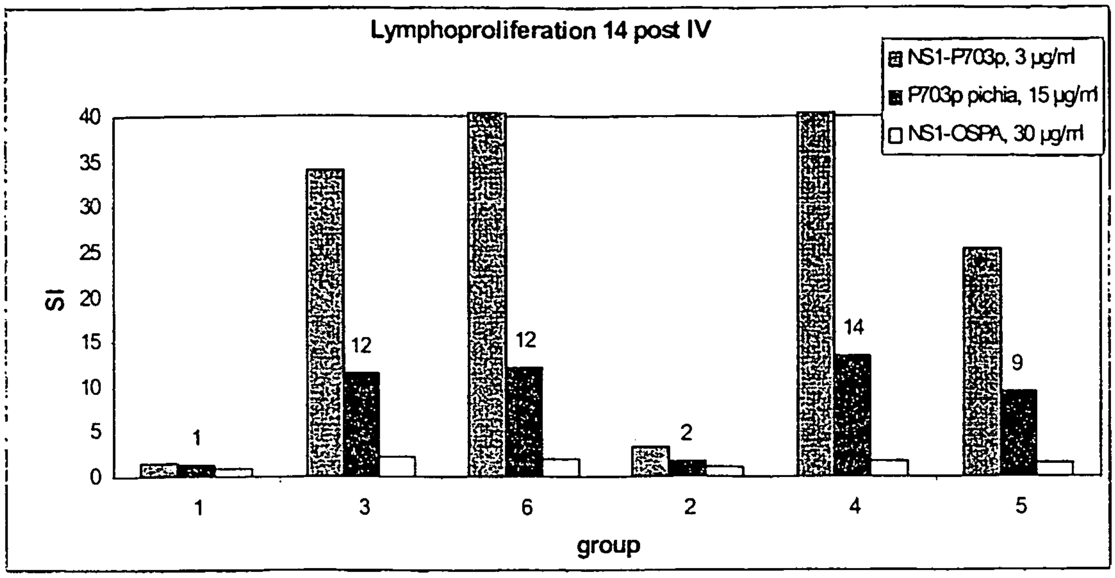

FIGURE 12

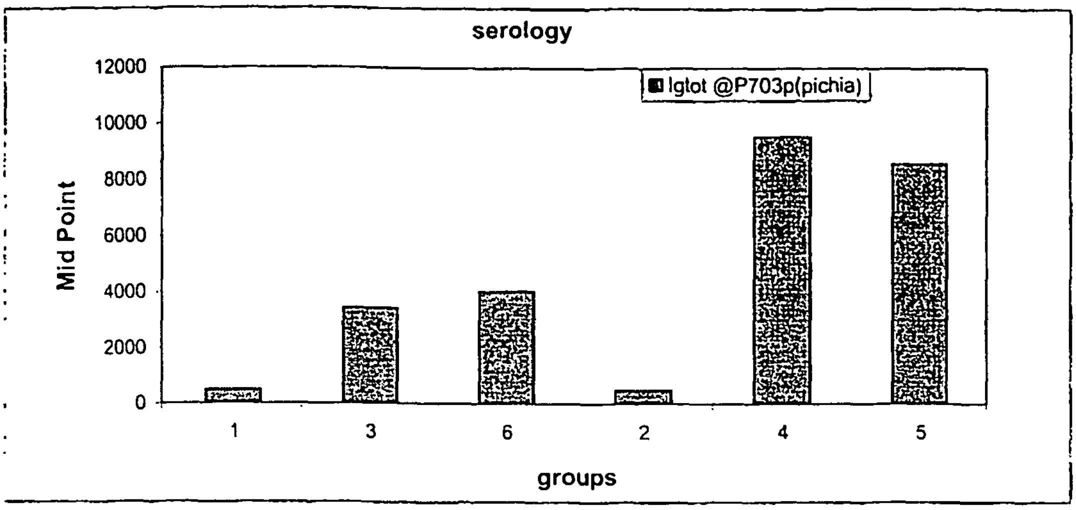
serology
Igtot @P703p(pichia)
Mid Point
12000
10000
8000
6000
4000
2000
0
1
3
6
2
4
5
groups

FIGURE 13 - Anti- NS1-P703 Antibody titres induced by vaccination

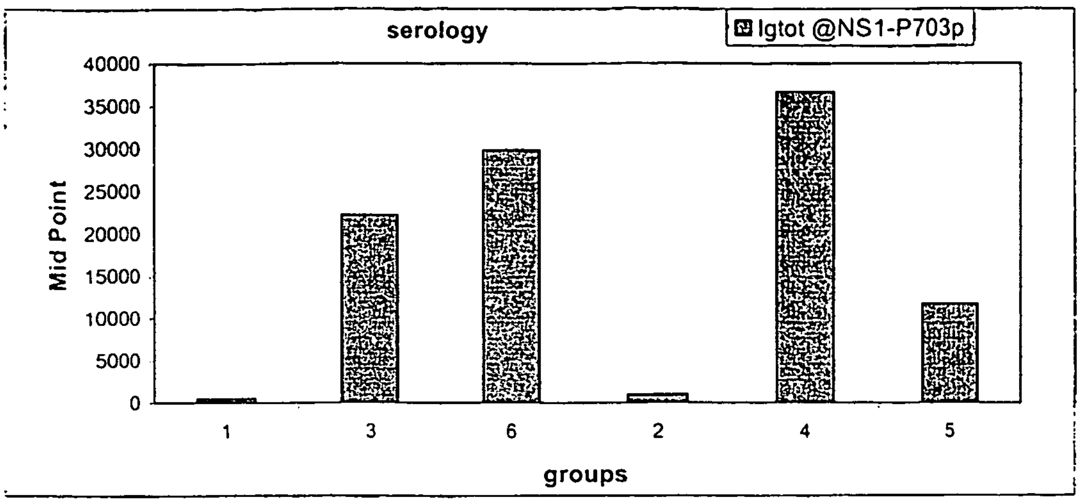

FIGURE 14 - Anti-NS1-P703P Isotype distribution induced by vaccines

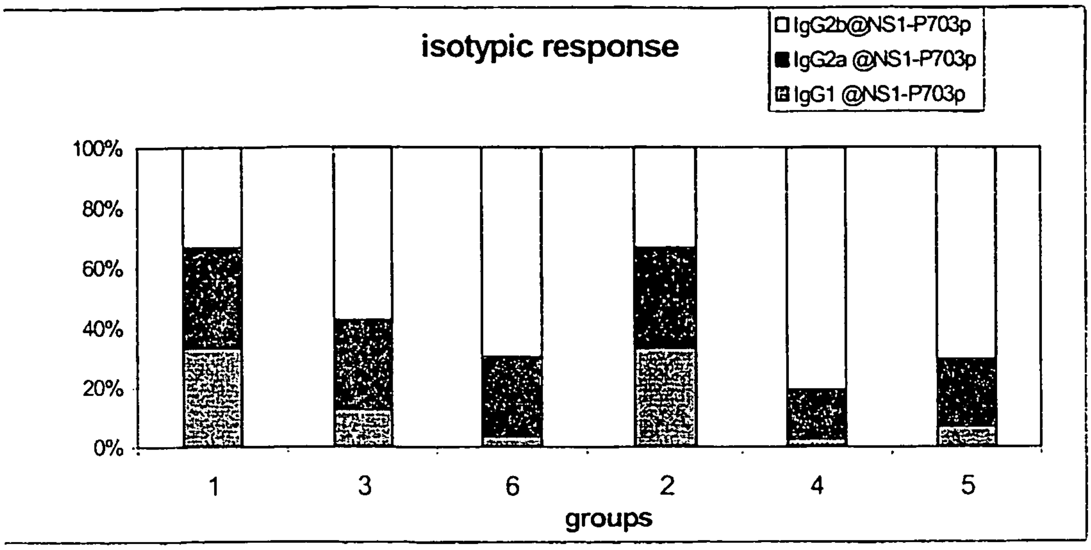

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9602555 A
- EP 468520 A
- WO 9816247 A
- GB 2122204 B
- WO 9421292 A
- WO 9843670 A2
- GB 2220211 A
- US 4912094 A
- WO 9801139 A
- US 6005099 A
- EP 0729473 B1
- EP 0549074 B1
- EP 0761231 B
- WO 9517210 A
- US 5057540 A
- EP 0362279 B1
- EP 0109942 B1, Morein, B.
- WO 9611711 A
- EP 0362279 A
- WO 9910008 A
- WO 9633739 A
- WO 9856415 A
- WO 9940188 A
- WO 9953061 A
- WO 9118926 A
- WO 9837814 A
- WO 9850567 A
- US 5654140 A
- US 5801005 A
- WO 0044899 A
- US 5666153 A
- US 5278302 A
- WO 9526204 A
- US 3238190 A, Erbring
- WO 9815287 A
- GB 9822712 A
- EP 0399843 B
- WO 9856414 A
- WO 9912565 A
- WO 9911241 A
- US 5422109 A
- EP 0480982 B2
- US 5424067 A
- EP 0480981 B
- EP 0382271 B1
- WO 9927961 A
- US 4596556 A
- US 5993412 A
- WO 9748440 A
- WO 9828037 A
- WO 9820734 A
- EP 0671948 B1
- EP 0689454 B1
- EP 0822831 A
- EP 0822831 B1


### Non-patent literature cited in the description


- **DAVIS et al.** *J.Immunol,* 1998, vol. 160 (2), 870-876
- **MCCLUSKIE ; DAVIS.** *J.Immunol.,* 1998, vol. 161 (9), 4463-6
- **WOOLDRIGE et al.** *macrophages,* 1977, vol. 89 (8
- **BRAZOLOT-MILLAN et al.** *Proc.Natl.Acad.Sci., USA,* 1998, vol. 95 (26), 15553-8
- **RIBI et al.** Immunology and Immunopharmacology of bacterial endotoxins. Plenum Publ. Corp, 1986, 407-419
- **HILGERS et al.** *Int.Arch.Allergy.Immunol.,* 1986, vol. 79 (4), 392-6
- **HILGERS et al.** *Immunology,* 1987, vol. 60 (1), 141-6
- **KENSIL, C. R.** Saponins as vaccine adjuvants. *Crit Rev Ther Drug Carrier Syst,* 1996, vol. 12 (1-2), 1-55
- **KENSIL et al.** *J. Immunology,* 1991, vol. 146, 431-437
- **BOMFORD et al.** *Vaccine,* 1992, vol. 10 (9), 572-577
- **GIZURARSON et al.** *Vaccine Research,* 1994, vol. 3, 23-29
- **MAHARAJ et al.** *Can.J.Microbiol,* 1986, vol. 32 (5), 414-20
- **CHAVALI ; CAMPBELL.** *Immunobiology,* vol. 174 (3), 347-59
- **MCI MOWAT et al.** *Immunology,* 1991, vol. 72, 317-322

- **MCI MOWAT ; DONACHIE.** *Immunology Today,* vol. 12, 383-385
- **SUMINO et al.** *J. Virol.,* 1998, vol. 72 (6), 4931-9
- **LACAILLE-DUBOIS, M ; WAGNER H.** A review of the biological and pharmacological activities of saponins. *Phytomedicine,* 1996, vol. 2, 363-386
- **ROBBINS ; KAWAKAMI.** *Current Opinions in Immunology,* 1996, vol. 8, 628-636
- **VAN DEN EYNDE et al.** *International Journal of Clinical & Laboratory Research,* 1997
- **CORREALE et al.** *Journal of the National Cancer Institute 89,* 1997, 293
- *Gene,* 1986, vol. 43, 265-272
- *Biotechnology,* 1992, vol. 10, 795-798
- **SALOMON et al.** *Bioessays,* vol. 199 (21), 61-70
- **FIEDLER.** *Arzneimittel-Forsch.,* 1953, vol. 4, 213
- **YOSHIKAWA M et al.** *Chem Pharm Bull (Tokyo,* August 1996, vol. 44 (8), 1454-1464
- **GISVOLD et al.** *J.Am.Pharm.,Assoc.,* 1934, vol. 23, 664
- **RUHENSTROTH-BAUER.** *Physiol.Chem.,* 1955, vol. 301, 621
- Dorland's Illustrated Medical Dictionary. W.B. Sanders Company, 1974
- **VOLLER et al.** New Trends and Developments in Vaccines. University Park Press, 1978
- **FERGUSON et al.** *Proc. Natl. Acad. Sci. USA,* 1999, vol. 96, 3114-3119